(19) Europäisches Patentamt
European Patent Office
Office européen des brevets

(11) **EP 4 192 457 B1**

(12) **EUROPEAN PATENT SPECIFICATION**

(45) Date of publication and mention
of the grant of the patent:
**25.03.2026 Bulletin 2026/13**

(21) Application number: **21853813.0**

(22) Date of filing: **06.08.2021**

(51) International Patent Classification (IPC):
*A61K 31/4535* (2006.01)    *A61K 31/167* (2006.01)
*A61K 45/06* (2006.01)    *A61K 9/00* (2006.01)
*A61K 31/192* (2006.01)    *A61K 31/405* (2006.01)
*A61P 31/14* (2006.01)

(52) Cooperative Patent Classification (CPC):
(C-Sets available)
**A61K 45/06; A61K 9/0078; A61K 31/192;
A61K 31/405; A61K 31/4535; A61P 31/14;**
A61K 9/0053    (Cont.)

(86) International application number:
**PCT/US2021/044897**

(87) International publication number:
**WO 2022/032069 (10.02.2022 Gazette 2022/06)**

(54) **COMPOSITIONS COMPRISING KETOTIFEN AND A NON-STEROIDAL ANTI-INFLAMMATORY DRUG SELECTED FROM NAPROXEN OR INDOMETHACIN FOR USE IN THE TREATMENT OF A BETA CORONAVIRUS INFECTION**

ZUSAMMENSETZUNGEN ENTHALTEND KETOTIFEN UND EINEN NICHTSTEROIDALEN ENTZÜNDUNGSHEMMER AUSGEWÄHLT AUS NAPROXEN ODER INDOMETHACIN ZUR VERWENDUNG BEI DER BEHANDLUNG EINER BETA-CORONAVIRUS-INFEKTION

COMPOSITIONS COMPRENANT DU KÉTOTIFÈNE ET UN MÉDICAMENT ANTI-INFLAMMATOIRE NON STÉROÏDIEN CHOISI PARMI LE NAPROXÈNE OU L'INDOMÉTHACINE POUR SON UTILISATION DANS LE TRAITEMENT D'UNE INFECTION À CORONAVIRUS BÊTA

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.08.2020 US 202063062138 P**

(43) Date of publication of application:
**14.06.2023 Bulletin 2023/24**

(73) Proprietor: **Sen-Jam Pharmaceutical LLC
Huntington, New York 11743 (US)**

(72) Inventors:
• **IVERSEN, Jacqueline M.
Lloyd Harbor, NY 11743 (US)**
• **DAHL, Thomas A.
Guilford, CT 06437 (US)**

(74) Representative: **Grund, Martin et al
Grund Intellectual Property Group
Patentanwälte und Solicitor PartG mbB
Steinsdorfstraße 2
80538 München (DE)**

(56) References cited:
**WO-A1-2019/046201    US-A1- 2018 360 811
US-A1- 2019 060 297**

• **JIM�NEZ-ALBERTO ALICIA ET AL: "Virtual screening of approved drugs as potential SARS-CoV-2 main protease inhibitors",
COMPUTATIONAL BIOLOGY AND CHEMISTRY, ELSEVIER, AMSTERDAM, NL, vol. 88, 25 June 2020 (2020-06-25), XP086476753, ISSN: 1476-9271, [retrieved on 20200625], DOI: 10.1016/ J.COMPBIOLCHEM.2020.107325**

EP 4 192 457 B1

• NAPOLI PIETRO EMANUELE ET AL: "A Panel of Broad-Spectrum Antivirals in Topical Ophthalmic Medications from the Drug Repurposing Approach during and after the Coronavirus Disease 2019 Era", vol. 9, no. 8, 30 July 2020 (2020-07-30), XP055906678, Retrieved from the Internet <URL:https://www.ncbi.nlm.nih.gov/pmc/articles/PMC7463888/pdf/jcm-09-02441.pdf> DOI: 10.3390/jcm9082441
• NARENDRANATHAN: "Ketotifen in prevention of indomethacin-induced gastropathy", INDIAN JOURNAL OF GASTROENTEROLOGY, 1 January 1999 (1999-01-01), pages 1 - 2, XP093192393
• NAPOLI PIETRO EMANUELE, MANGONI LORENZO, GENTILE PIETRO, BRAGHIROLI MIRCO, FOSSARELLO MAURIZIO: "A Panel of Broad-Spectrum Antivirals in Topical Ophthalmic Medications from the Drug Repurposing Approach during and after the Coronavirus Disease 2019 Era", JOURNAL OF CLINICAL MEDICINE, vol. 9, no. 8, 30 July 2020 (2020-07-30), XP055906678, DOI: 10.3390/jcm9082441

(52) Cooperative Patent Classification (CPC): (Cont.)

C-Sets
**A61K 31/192, A61K 2300/00;
A61K 31/405, A61K 2300/00;
A61K 31/4535, A61K 2300/00**

**Description**

BACKGROUND

Coronaviruses are a large family of viruses that cause illness ranging from the common cold to more severe diseases such as Middle East Respiratory Syndrome (MERS-CoV), Severe Acute Respiratory Syndrome (SARS-CoV) and Coronavirus disease 2019 (COVID-19). Coronaviruses are zoonotic, meaning that they are transmitted between animals and people. COVID-19 is an infectious disease caused by the severe acute respiratory syndrome coronavirus 2 (SARS-CoV-2). SARS-CoV-2 virus is an enveloped, positive-sense, single-stranded RNA beta-coronavirus. The disease has spread globally since 2019.

SARS-CoV-2 is spread via respiratory droplets, often produced during coughing. Time from exposure to onset of symptoms is generally between two and 14 days, with an average of five days. The standard method of diagnosis is by reverse transcription polymerase chain reaction (rRT-PCR) from a nasopharyngeal swab or sputum sample. Antibody assays can also be used, using a blood serum sample. The infection can also be diagnosed from a combination of symptoms, risk factors and a chest CT scan showing features of pneumonia.

COVID -19 is a novel, poorly understood disease with limited effective treatments. Common symptoms of COVID-19 include fever, cough and shortness of breath. Muscle pain, sputum production and sore throat are some of the less common symptoms. While the majority of cases result in mild symptoms, some progress to pneumonia, severe acute respiratory syndrome, multi-organ failure and even death. The case fatality rate is estimated at between 1% and 5% and varies by age and other health conditions. The disease is more likely to occur in people whose immune system is impaired due to age, immunosuppressive therapy, chronic disease, psychological stress, or other factors.

The literature reports on studies directed to find approved drugs as potential anti-SARS-CoV-2 agents (Jimenez-Alberto Alicia et al., "Virtual screening of approved drugs as potential SARS-CoV-2 main protease inhibitors", 2020, Computational Biology and Chem., Elsevier, Amsterdam, NL, vol. 88; and , Napoli Pietro Emanuele et al., "A Panel of Broad-Spectrum Antivirals in Topical Ophthalmic Medications from the Drug Repurposing Approach during and after the Coronavirus Disease 2019 Era", 2020, Journal of Clinical Medicine, vol. 9, no. 8).

Accordingly, a need exists for an effective and safe method of preventing or treating infection of beta-coronaviruses, such as SARS-CoV-2, and/or reducing symptoms of associated disease (e.g., COVID-19).

SUMMARY

The present disclosure relates to methods and compositions for treating or preventing a beta-coronavirus infection, or for reducing symptoms of a disease caused by a beta coronavirus infection in a subject, including SARS-CoV-2 and COVID-19. In various embodiments, the composition and methods involve a combination of a non-steroidal anti-inflammatory drug (NSAID) and ketotifen, wherein the NSAID is selected from naproxen or indomethacin. Naproxen and/or indomethacin are combined with ketotifen for therapy in a single unit dose or separate compositions, and the combination is surprisingly and unexpectedly effective to reduce SARS-CoV-2 viral load and/or reduce COVID-19 symptoms.

In various embodiments, the methods comprise the administration of one or more pharmaceutical compositions to a mammal, typically a human, in an amount which is effective for treating or preventing diseases caused by the SARS-CoV-2, and/or reducing the symptoms thereof. Examples of diseases caused by SARS-CoV-2 include Coronavirus Disease 2019 (i.e., COVID-19).

In embodiments, the disclosure provides for treatment of a subject who is at risk of developing a disease caused by SARS-CoV-2 (or other beta-coronavirus). In some embodiments, a subject is at risk of developing a disease caused by SARS-CoV-2 due to age, an immunocompromised status, or due to one or more pre-existing conditions.

In various embodiments, therapy can be initiated before the onset of COVID-19 symptoms or after the onset of symptoms. In some embodiments, therapy is initiated soon after the first appearance of symptoms (i.e., as soon as possible after the first appearance of symptoms). In some embodiments, a subject according to the disclosure is asymptomatic, but tests positive for SARS-CoV-2 (or other beta-coronavirus). In some embodiments, therapy according to the disclosure is initiated as soon it is first suspected that the individual has an infection or disease caused by SARS-CoV-2 (or other beta-coronavirus), but before the onset of symptom(s) of the disease. In various embodiments, the subject has one or more risk factors for COVID-19. In various embodiments, the subject is experiencing one or more symptoms selected from congestion, cough, fever, loss of taste, loss of smell, muscle pain, joint pain (myalgia), fatigue, sore throat, headache, myocarditis, coagulopathy, renal injury or failure, liver damage or failure, neurological impairment, hypoxia, pneumonia with or without hypoxia, acute respiratory syndrome, or multi-organ failure.

In various embodiments, the present invention provides for administration of one or more pharmaceutical compositions to a human subject, in an amount which is effective to treat or prevent a beta-coronavirus infection or associated disease in a subject. The NSAID, selected from naproxen or indomethacin, and ketotifen may be administered as a single unit dose (composition), or in separate compositions. In some embodiments, a method of the disclosure comprises administering an effective amount of indomethacin and ketotifen. In certain embodiments, indomethacin and ketotifen are administered at least daily, and the daily dose of indomethacin is about 20 mg to about 300 mg, about 40 mg to about 200 mg, or about 75 mg to about 150 mg. In various embodiments, a daily dose of ketotifen is about 1 mg to about 10 mg, or about 1 mg to about 6 mg, or about 2 mg to about 5 mg, or about 3 mg to about 4 mg. In some embodiments, a method of the disclosure comprises administering naproxen and ketotifen. In certain embodiments, naproxen and ketotifen are administered at least daily, and the daily dose of naproxen is about 100 mg to about 1600 mg, about 200 mg to about 1400 mg, about 400 mg to about 1200 mg, about 600 mg to about 1000 mg, or about 700 mg to about 900 mg. In various embodiments, a daily dose of ketotifen is about 1 mg to about 10 mg, or about 1 mg to about 6 mg, or about 2 mg to about 5 mg, or about 3 mg to about 4 mg. In certain embodiments, a unit dose of the disclosure comprises 50 mg indomethacin and 1 mg ketotifen, and the unit dose is administered 2 or 3 times per day.

In some embodiments, the disclosure provides pharmaceutical compositions that consist essentially of the NSAID indomethacin and/or naproxen, and ketotifen as active pharmaceutical ingredients. In some embodiments, the pharmaceutical composition consists of the NSAID indomethacin and/or naproxen, and ketotifen as active pharmaceutical ingredients. In these embodiments, the composition may comprise inactive ingredients, such as pharmaceutical excipients, sweetening agent(s), flavoring agent(s), taste masking ingredient(s), diluent(s), alum, stabilizer(s), buffer(s), coloring agent(s), breath neutralizer(s) (e.g., peppermint or menthol scents), emulsifying agent(s), suspending agent(s), and agents to control delivery. The compositions in some embodiments may have additional ingredients (including active ingredients) that do not affect the basic and novel characteristics of the composition.

In accordance with embodiments of the disclosure, the pharmaceutical composition(s) can be administered orally, intravenously, intramuscularly, subcutaneously, transdermally, by inhalation, or intranasally. In further embodiments, a composition comprising ketotifen and an NSAID selected from naproxen or indomethacin are combined in the form of a tablet, a capsule, a lozenge or chewing gum.

Other aspects and embodiments of the present disclosure will be apparent from the following detailed description.

Any references in the description to methods of treatment refer to the compounds, pharmaceutical compositions and medicaments of the invention for use in a method of treatment of the human body by therapy.

Any references to non-steroidal anti-inflammatory drug (NSAID) refer to naproxen or indomethacin.

## BRIEF DESCRIPTION OF THE DRAWINGS

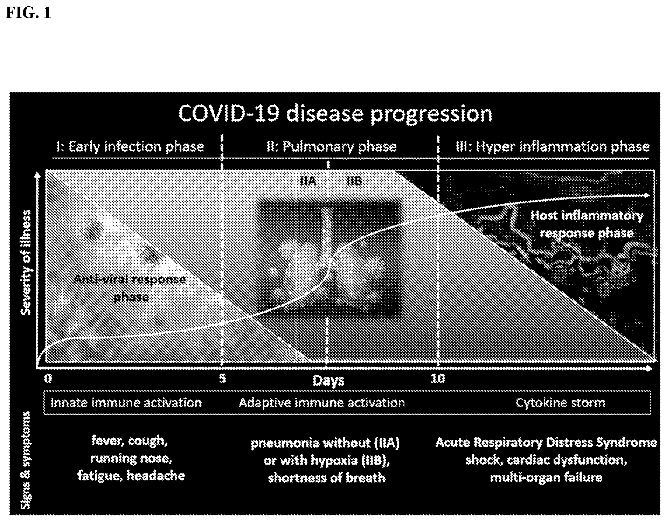

FIG. 1 shows COVID-19 disease progression: Early infection phase involving activation of the innate immune system, pulmonary phase involving activation of the adaptive immune system, and hyperinflammation phase involving cytokine storm.

FIG. 2 shows dose-response curves for naproxen and ketotifen, alone and in combination. $EC_{50}$ is the effective concentration of product, i.e., the concentration at which virus infection is inhibited by 50 percent.

FIG. 3 shows dose-response curves for indomethacin and ketotifen, alone and in combination.

## DETAILED DESCRIPTION

The present disclosure relates to methods and compositions for treating or preventing a beta-coronavirus infection, or for reducing symptoms of a disease caused by a beta coronavirus infection in a subject, including SARS-CoV-2 and COVID-19. In various embodiments, the composition and methods involve a combination of a non-steroidal anti-inflammatory drug (NSAID) and ketotifen. Naproxen and/or indomethacin are combined with ketotifen for therapy in a single unit dose or separate compositions, and the combination is surprisingly and unexpectedly effective to reduce SARS-CoV-2 viral load and/or reduce COVID-19 symptoms.

Accordingly, the disclosure provides therapeutic and prophylactic pharmaceutical compositions for diseases caused by beta-coronaviruses, including SARS-CoV-2. The SARS-CoV-2 virus is an enveloped, positive-sense, single-stranded RNA beta-coronavirus. The methods and compositions of the disclosure are effective to treat, reduce, or prevent symptoms of a disease caused by distinct genetic variants of SARS-CoV-2, including the B.1.1.7 (i.e., Alpha), B.1.351 (i.e., Beta), P.1 (i.e., Gamma), B.1.617.2 (i.e., Delta), and C.37 (i.e., Lambda) variants of SARS-CoV-2.

In various embodiments, the methods comprise the administration of one or more pharmaceutical compositions to a mammal, typically a human, in an amount which is effective for treating or preventing diseases caused by the SARS-CoV-2, and/or reducing the symptoms thereof. Examples of diseases caused by SARS-CoV-2 include Coronavirus Disease 2019 (i.e., COVID-19). The clinical progression of COVID-19 ranges from asymptomatic carriage to fulminant cytokine storm with respiratory failure, multi-organ dysfunction and ultimately death. Early infection (or Stage I), is characterized by activation of the innate immune system, and exhibits increased cytokine and chemokine production. This stage is the incubation period when SARS-CoV-2 multiplies and establishes residence in the host, primarily focusing on the respiratory system. Symptoms reported during early infection are usually mild and often non-specific, including fever, cough, diarrhea, and headache. COVID-19 infection may progress to a pulmonary phase (or "Stage II"), which can be divided into two distinct parts: pneumonia without hypoxia (Stage IIA) or pneumonia with hypoxia (Stage IIB). During Stage IIB, symptoms worsen, and patients will likely require hospitalization and oxygen supplementation. Some infections progress to a Stage III, which manifests as an extrapulmonary systemic hyper inflammation syndrome, driven by the cytokine storm. Stage III is characterized by exuberant inflammation resulting from high circulating cytokines such as interleukin (IL)-6 and tumor necrosis factor alpha (TNF-$\alpha$). These inflammatory mediators are responsible for the multi-organ damage, including acute respiratory distress syndrome (ARDS) and cardiac and renal dysfunction. In general, the prognosis and recovery from Stage III are poor.

Mast cells can be strongly activated during SARS-CoV-2 infection, producing damage to the lung tissue, with hemorrhaging visible on the lungs and fluid accumulation in the lungs, as well as producing other COVID-19 symptoms. Traditionally, mast cell stabilizers such as ketotifen have been in used in humans for the treatment of allergy and asthma, and these drugs are thought to act by preventing degranulation of mast cells. Thus, in various embodiments, the invention involves the combination of a mast cell stabilizer and an NSAID for preventing or treating a coronavirus infection or associated disease. An exemplary mast cell stabilizer is ketotifen.

The disclosure provides for treatment of a subject who is at risk of developing a disease caused by SARS-CoV-2 (or other beta-coronavirus). As used herein, a subject at risk of developing a disease caused by SARS-CoV-2 is not infected with SARS-CoV-2 or other beta-coronavirus (i.e., tests negative for SARS-CoV-2), or is not otherwise known to be infected with SARS-CoV-2. In some embodiments, a subject is at risk of developing a disease caused by SARS-CoV-2 has come into close contact with one or more SARS-CoV-2 positive individuals. As used herein, close contact means, for example, someone who has been, or is suspected of having been, within 6 feet of an individual infected with SARS-CoV-2 for a total of at least 15 minutes, or for a total of at least one hour, or for over a 24-hour period. Close contact with someone with confirmed COVID-19 includes, without limitation, a subject who came into contact, or is suspected of having come into contact, with one or more SARS-CoV-2 positive individuals in a daycare center, airplane, restaurant, school, healthcare setting (such as a physicians office or a hospital). A close contact also includes a subject who is a health care worker or first responder (such as, without limitation, a law enforcement officer, emergency medical technician, nurse, physician, or firefighter) who has come into contact with someone who is, or is suspected of being, infected with SARS-CoV-2.

A subject might be at risk of developing a disease caused by SARS-CoV-2 due to age, an immunocompromised status, or due to one or more pre-existing conditions. For example, subjects who are at risk may be elderly (i.e., over age 65 years old), or may be immunocompromised due to a disease (genetic or acquired disease) or due to therapy with an immunosuppressant. Subjects at risk of developing a beta-coronavirus associated disease may also include those with pre-existing conditions, such as a chronic health condition such as, without limitation, one or more of: obesity; diabetes mellitus (type 1 or 2); AIDS; a chronic lung condition such as asthma, COPD, pulmonary fibrosis, cystic fibrosis, pulmonary hypertension, emphysema, and bronchitis; neutrophilia; history of myocardial infarction or stroke; coronary artery disease; cardiomyopathy; dementia or Alzheimer's disease; chronic kidney or liver disease; organ transplantation; and auto-immune disease (e.g., SLE or RA). In some embodiments, the subject has a history of smoking tobacco.

Subjects who have weakened or compromised immune systems include, without limitation, cancer patients, in particular those who are undergoing chemotherapy, radiation therapy, or immunotherapy; individuals with organ or tissue transplants; individuals who take pharmaceuticals to suppress their immune response (e.g., corticosteroids, cyclosporine, tacrolimus); individuals with HIV or AIDS; diabetics; individuals recovering from an illness, surgery or trauma; and individuals with poor nutrition.

Prophylactic administration of the NSAID and ketotifen is also suited for individuals who cannot tolerate (or cannot benefit from) a vaccine. For example, it is known that many elderly individuals do not react efficiently to vaccines. In embodiments, prophylactic administration of a pharmaceutical composition according to the disclosure comprises administration before the subject is infected by SARS-CoV-2, or before SARS-CoV-2 is detected in the subject.

In some embodiments, the therapy according to this disclosure is provided at the time of vaccination (for one or a plurality of days), to inhibit inflammatory symptoms associated with the beta-coronavirus spike protein contained in or encoded by the vaccine.

In various embodiments, therapy can be initiated before the onset of symptoms or after the onset of symptoms. In some embodiments, therapy is initiated soon after the first appearance of symptoms (i.e., as soon as possible after the first appearance of symptoms). In some embodiments, a subject according to the disclosure is asymptomatic, but tests positive for SARS-CoV-2 (or other beta-coronavirus). As used herein, testing for SARS-CoV-2 (or other beta-coronavirus) includes, without limitation, a genetic test (e.g. a PCR, or nucleic acid amplification, based test), an antigen test, or an antibody (i.e. serology) test. In some embodiments, therapy according to the disclosure is initiated as soon it is first suspected that the individual has an infection or disease caused by SARS-CoV-2 (or other beta-coronavirus), but before the onset of a symptom of the disease. In various embodiments, the subject has one or more risk factors for COVID-19 as already described.

In various embodiments, the subject is experiencing one or more symptoms selected from congestion, cough, fever, loss of taste, loss of smell, muscle pain, joint pain (myalgia), fatigue, sore throat, headache, myocarditis, coagulopathy, renal injury or failure, liver damage or failure, neurological impairment, hypoxia, pneumonia with or without hypoxia, acute respiratory syndrome, or organ failure.

In some embodiments, therapy according to this disclosure is initiated during a beta-coronavirus or SARS-CoV-2 incubation period, when the virus multiplies and establishes residence in the subject, primarily in the respiratory system of the subject. Symptoms during this stage are usually mild and often non-specific, and may include including fever, cough, sore throat, diarrhea, loss of taste or smell, fatigue, and headache.

In some embodiments, therapy according to this disclosure is initiated during the pulmonary phase, which can include hypoxia, and may require oxygen supplementation.

In still other embodiments, therapy according to this disclosure is initiated in a subject experiencing extrapulmonary systemic hyper inflammation syndrome. For example, the subject may exhibit exuberant inflammation resulting from high circulating cytokines such as interleukin (IL)-6 and tumor necrosis factor alpha (TNF-α). In some embodiments, the subject may be experiencing acute respiratory syndrome and/or multi-organ failure.

In some embodiments, a therapy according to the disclosure is administered to a subject with early symptoms of COVID-19. Examples of early COVID-19 symptoms include, without limitation, respiratory symptoms (without substantial hypoxia), congestion, cough, fever, loss of taste or smell (anosmia), muscle pain, joint pain (myalgia), fatigue, sore throat, or headache.

In some embodiments, a therapy according to this disclosure is administered to a subject with symptoms of long COVID, or the therapy according to this disclosure prevents or reduces the likelihood of long COVID. As used herein, long COVID refers to a range of new or ongoing symptoms that can last weeks or months after a subject is first infected with SARS-CoV-2 or experiences symptoms of COVID-19. Long COVID can occur in any subject who has had COVID-19, even if the illness was mild, or if they had no initial symptoms. Long COVID symptoms include one or more of difficulty breathing or shortness of breath, tiredness or fatigue, symptoms aggravated after physical or mental activities, difficulty thinking or concentrating (sometimes referred to as "brain fog"), cough, chest or stomach pain, headache, fast-beating or pounding heart (i,e,, heart palpitations), joint or muscle pain, diarrhea, sleep problems, fever, dizziness on standing (lightheadedness), rash, mood changes, change in smell or taste, changes in menstrual cycles.

The NSAID comprises naproxen (6-methoxy-α-methyl-2-naphthaleneacetic acid) and/or indomethacin (2-{1-[(4-chlorophenyl)-carbonyl]-5-methoxy-2-methyl-1*H*-indol-3-yl}acetic acid). In some embodiments, the disclosure provides a pharmaceutical composition comprising indomethacin, naproxen and ketotifen.

As used herein, the NSAID or ketotifen include all pharmaceutically acceptable versions thereof, including, for example, all pharmaceutically acceptable salts thereof, stereoisomers and/or any mixtures thereof, all pharmaceutically acceptable zwitterions and/or any mixtures thereof, all pharmaceutically acceptable polymorphic forms and/or any mixtures thereof, and all pharmaceutically acceptable complexes (including solvates) and/or any mixtures thereof. Salts include their racemates, enantiomers, or any mixtures thereof. Particularly suitable salts comprise alkali-metal salts (e.g., sodium and/or potassium salts), alkaline earth metal salts (e.g., magnesium and/or calcium salts), aluminum salts, ammonium salts, salts of suitable organic bases (e.g., salts of alkylamines and/or-methyl-D-glutamine), salts of amino acids ( e.g., salts of arginine and/or lysine). Salts also include all enantiomeric salts formed with pharmaceutically acceptable chiral acids and/or bases and/or any mixtures of enantiomers of such salts (e.g., (+) tartrates, (-) tartrates and/or any mixtures thereof including racemic mixtures). Examples of typical salts include naproxen sodium, indomethacin sodium and ketotifen fumarate.

As used herein, "treating" a disease caused by SARS-CoV-2 means reducing, eliminating, or preventing at least one symptom of the disease, or reducing or eliminating the presence or activity of SARS-CoV-2 infection in the subject. Treatment includes the prevention and/or attenuation of the symptoms and/or morbidity associated with the disease. A treatment is effective if it prevents or reduces a symptom associated with a disease caused by a beta-coronavirus or SARS-CoV-2, especially reducing or preventing congestion, cough, fever, loss of taste, loss of smell, muscle pain, joint pain (myalgia), fatigue, sore throat, headache, hypoxia, pneumonia with or without hypoxia, severe acute respiratory syndrome, or organ failure. Further, a treatment is effective if it shortens the duration or severity of symptoms of the disease.

As used herein, "preventing" a disease caused by SARS-CoV-2 means preventing a subject from being infected with SARS-CoV-2, or preventing a subject infected with SARS-CoV-2 from developing disease, or from disease progression, such as by reducing or eliminating the presence or activity of SARS-CoV-2 in the subject.

The methods of the present invention comprise the administration of one or more pharmaceutical compositions to a human subject, in an amount which is effective to treat a beta-coronavirus infection or associated disease in a subject. The NSAID and ketotifen may be administered as a single unit dose (composition), or in separate compositions. Administration includes administration by a physician or by self-administration. The actual amounts of the active agents in a specific case can vary according to the particular compositions formulated, the mode of application, the particular sites of application, and the subject being treated (e.g., age, gender, size, tolerance to drug, etc.).

In some embodiments, a method of the disclosure comprises administering an effective amount of indomethacin and ketotifen. In certain embodiments, indomethacin and ketotifen are administered at least daily, and the daily dose of indomethacin is about 20 mg to about 300 mg, about 40 mg to about 200 mg, or about 75 mg to about 150 mg. In various embodiments, a daily dose of ketotifen is about 1 mg to about 10 mg, or about 1 mg to about 6 mg, or about 2 mg to about 5 mg, or about 3 mg to about 4 mg.

In some embodiments, a method of the disclosure comprises administering naproxen and ketotifen. In certain embodiments, naproxen and ketotifen are administered at least daily, and the daily dose of naproxen is about 100 mg to about 1600 mg, about 200 mg to about 1400 mg, about 400 mg to about 1200 mg, about 600 mg to about 1000 mg, or about 700 mg to about 900 mg. In various embodiments, a daily dose of ketotifen is about 1 mg to about 10 mg, or about 1 mg to about 6 mg, or about 2 mg to about 5 mg, or about 3 mg to about 4 mg.

In certain embodiments, a method of the disclosure comprises administering an effective amount of a pharmaceutical composition comprising a dose of indomethacin of about 20 mg to about 300 mg, about 40 mg to about 200 mg, or about 75 mg to about 150 mg, and a dose of ketotifen of about 1 mg to about 6 mg, about 2 mg to about 5 mg, or about 3 mg to about 4 mg. In some embodiments, the disclosure comprises administering a pharmaceutical composition comprising ketotifen and at least one NSAID combined in unit dose. In certain embodiments, a unit dose of the disclosure comprises about 50 mg indomethacin and about 1 mg ketotifen, and the unit dose is administered 2 or 3 times per day.

In some embodiments, the disclosure provides pharmaceutical compositions that consist essentially of the NSAID (e.g., indomethacin and/or naproxen) and ketotifen as active pharmaceutical ingredients. In some embodiments, the pharmaceutical composition consists of the NSAID (indomethacin and/or naproxen) and ketotifen as active pharmaceutical ingredients. In these embodiments, the composition may comprise inactive ingredients, such as pharmaceutical excipients, sweetening agent(s), flavoring agent(s), taste masking ingredient(s), diluent(s), alum, stabilizer(s), buffer(s), coloring agent(s), breath neutralizer(s) (e.g., peppermint or menthol scents), emulsifying agent(s), suspending agent(s), and agents to control delivery, for example. The compositions in some embodiments may have additional ingredients (including active ingredients) that do not affect the basic and novel characteristics of the composition. Such ingredients, which can be specifically excluded in some embodiments, include: cyclooxygenase-2-selective inhibitors (i.e., COX-2-selective inhibitors) or prodrugs thereof, an adrenergic agent, an anticholinergic agent, zinc (e.g., zinc acetate, zinc gluconate, zinc sulfate), pleconaril, a phosphodiesterase type 4 modulator, recombinant human uteroglobin, IL-9 antagonists or IL-8 antagonists (including blocking monoclonal antibodies or peptides), calcium glycerophosphate, sedating antihistamines, decongestants, ginsenoside, aminoquinolones (e.g., chloroquine, hydroxychloroquine), protease inhibitors (e.g., lopinavir, ritonavir, nafamostat, and camostat), H2 receptor antagonist (e.g., famotidine), umifenovir, thiazolide (e.g., nitazoxanide), ivermectin, corticosteroids, tocilizumab, sarilumab, bevacizumab, selective serotonin-reuptake inhibitors (e.g., fluvoxamine), remdesivir, baricitinib, dexamethasone (glucocorticoid), aviptadil, and azithromycin.

In some embodiments, the disclosure comprises administering the NSAID (indomethacin and/or naproxen) and ketotifen at least once daily. In further embodiments, NSAID and ketotifen are administered from about once daily to about three times daily. In certain embodiments, the NSAID and ketotifen are administered for at least one week. In yet further embodiments, the NSAID and ketotifen are administered for at least two weeks. In still further embodiments, NSAID and ketotifen are administered for at least about three weeks, or about one month.

In some embodiments, the disclosure provides a pharmaceutical composition comprising an NSAID and ketotifen, wherein the composition further comprises a pharmaceutical carrier, a pharmaceutical excipient, a sweetening agent, a flavoring agent, a taste masking ingredient, a diluent, alum, a stabilizer, a buffer, a coloring agent, a breath neutralizer (e.g., peppermint or menthol scents), an emulsifying agent, a suspending agent, a nebulizing agent and combinations thereof.

Examples of daily amounts to be administered in the methods of the present invention follows. The daily amounts can be administered in one dose, or in multiple doses, typically, two doses. Quantities can be defined by ranges, and by lower and upper boundary ranges. Exemplary lower and upper boundaries follow, and each may be taken as a separate element.

Ketotifen may be administered from about 0.5 mg to about 10 mg: Examples of lower boundaries of this range include about 1 mg, about 3 mg, and about 4 mg. Examples of other upper boundaries of this range include about 5 mg, about 6 mg, and about 2.8 mg. An example of a typical dosage is about 1 mg - 4 mg/day, administered typically as 1-2 mg 2 times per day.

Naproxen from about 110 mg to about 1650 mg: Examples of other lower boundaries of this range include about 220 mg, about 320 mg, about 550 mg and about 720 mg. Examples of other upper boundaries of this range include about 880 mg, about 1000 mg, about 1300 mg and about 1500 mg. An example of a typical dosage is about 220 mg - 1100 mg/day, administered 2 times per day, more typically, 220 mg naproxen sodium 2 times per day or 550 mg naproxen sodium 2 times per day.

Indomethacin from about 25 mg to about 300 mg: Examples of other lower boundaries of this range include about 50 mg, about 75 mg, about 100 mg and about 125 mg. Examples of other upper boundaries of this range include about 150 mg, about 175 mg, about 200 mg and about 250 mg. An example of a typical dosage is about 50 - 150 mg/day, administered typically as 25 mg 3 times per day, or 50 mg 3 times per day.

In accordance with embodiments of the disclosure, the pharmaceutical composition(s) can be administered orally, intravenously, intramuscularly, subcutaneously, transdermally, by inhalation, or intranasally. In further embodiments, a composition comprising ketotifen and an NSAID (indomethacin and/or naproxen), are combined in in the form of a tablet, a capsule, a lozenge or chewing gum.

In still further embodiments, a composition of the disclosure is administered by a nebulizer, or by powder or solution aerosol (aerosol spray, mist, or powder), for local delivery to the lungs. Such local delivery is useful for subjects already experiencing pulmonary involvement.

In embodiments, compositions according to the disclosure can be administered orally by any method known in the art. For example, the compositions can be administered in the form of tablets, including, e.g., orally-dissolvable tablets, chewable tablets; capsules; lozenges; pills (e.g., pastilles, dragees); troches; elixirs; suspensions; syrups; wafers; chewing gum; strips; films (e.g., orally-dissolving thin films); soluble powders; effervescent compositions; and the like. In some embodiments, the composition is a tablet or capsule for oral delivery (e.g., enteral or buccal delivery).

In the case of tablets for oral use, carriers commonly used include lactose and corn starch, and lubricating agents such as magnesium stearate are commonly added. For oral administration in capsule form, useful carriers include lactose and com starch. Further examples of carriers and excipients include milk, sugar, certain types of clay, gelatin, stearic acid or salts thereof, calcium stearate, talc, vegetable fats or oils, gums and glycols.

When aqueous suspensions are used for oral administration, emulsifying and/or suspending agents are commonly added. In addition, sweetening and/or flavoring agents may be added to the oral compositions.

In additional embodiments, compositions according to the disclosure can be administered enterally or parenterally, e.g., intravenously; intramuscularly; subcutaneously, as injectable solutions or suspensions; intraperitoneally; sublingually; or rectally (e.g., by suppositories). For intramuscular, intraperitoneal, subcutaneous and intravenous use, sterile solutions of the pharmaceutically compositions can be employed, and the pH of the solutions can be suitably adjusted and buffered. For intravenous use, the total concentration of the solute(s) can be controlled in order to render the preparation isotonic.

In certain embodiments, the NSAID and ketotifen are provided as an orally dissolving tablet, with or without a taste masking ingredient, diluents, etc. Such tablet can be administered without water onto the tongue leading to immediate dissolution and is absorbed gastrointestinally or buccally. Orally dissolving tablets can be formulated by a number of techniques including compression and lyophilization, as would be known to a skilled artisan.

In some embodiments, the composition is a lozenge or troche comprising the NSAID and ketotifen with or without a taste masking ingredient, diluents, etc. Such lozenge/troche can be administered without water, and can slowly dissolve in the mouth, or can be swallowed or chewed. Such lozenges/troches can be formulated by compression, as would be known to a skilled artisan.

In various embodiments, the pharmaceutical composition is a controlled release composition. Controlled release drug delivery achieves a certain level of the drug over a particular period time. The level typically is measured by plasma concentration. Methods for controlled release of drugs are well known in the art. An example of a controlled release formulation is a capsule containing beadlets, wherein some of the beadlets dissolve instantaneously and some of the beadlets dissolve at delayed times dues to different types of beadlet coatings.

Other aspects and embodiments of the present disclosure will be apparent from the following illustrative examples.

EXAMPLES

The 2019 coronavirus infectious disease (COVID-19) is caused by infection with the new severe acute respiratory syndrome coronavirus (SARS-CoV-2). The treatment options for COVID-19 are limited. The purpose of these Examples was to demonstrate the effectiveness of ketotifen, naproxen and indomethacin, alone or in combination, in reducing SARS-CoV-2 replication. In addition, the cytotoxicity of the drugs was evaluated. The findings demonstrate that the combination of ketotifen with indomethacin or naproxen both reduce viral yield. Compared to ketotifen alone (60% inhibition at $EC_{50}$), an increase in percentage inhibition of SARS-CoV-2 to 79%, 83% and 93% was found when co-administered with 25, 50 and 100 $\mu$M indomethacin, respectively. Compared to ketotifen alone, an increase in percentage inhibition of SARS-CoV-2 to 68%, 68% and 92% was found when co-administered with 25, 50 and 100 $\mu$M naproxen, respectively. For both drug combinations the observations suggest an additive or synergistic effect, compared to administering the drugs alone. No cytotoxic effects were observed for the administered dosages of ketotifen, naproxen, and indomethacin.

Materials and Methods

Ketotifen, indomethacin, and naproxen were sourced from Sigma Aldrich and remdesivir from MedChemExpress. SARS-CoV-2 hCoV-19/Australia/VIC01/2020 was a gift from Melbourne's Peter Doherty Institute for Infection and Immunity. African Green Monkey Kidney (Vero E6) cells were obtained from ATCC (ATCC-CRL1586) (Noble Park North, VIC, Australia).

Virus stocks were expanded via passage in Vero E6 cells in growth media, which comprised Minimal Essential Medium without L-glutamine, supplemented with 1% (w/v) L-glutamine, 1.0 $\mu$g/mL of TPCK-Trypsin, 0.2% BSA, 1$\times$ Pen/Strep, and 1% Insulin Transferrin Selenium (ITS).

African Green Monkey Kidney (Vero E6) cells (ATCC-CRL1586) were subcultured to generate cell bank stocks in cell growth medium, which comprised of Minimal Essential Medium (MEM) without L-glutamine, supplemented with 10% (v/v) heat-inactivated Fetal Bovine Serum (FBS) and 1% (w/v) L-glutamine. Vero E6 cells were passaged for a maximum of 13 passages, after which a new working cell bank stock was retrieved from liquid nitrogen for further use.

*Cytopathic Effect (CPE) Assay*

The cytopathic effects of indomethacin, ketotifen, and naproxen were compared with remdesivir (positive control).

Vero E6 cells were seeded into 96-well plates at $2 \times 10^4$ cells/well in 100 $\mu$L seeding medium (MEM supplemented with 1% (w/v) L-glutamine, 2% FBS). Plates were incubated overnight at 37°C, 5% $CO_2$. Test compounds were prepared fresh on the day of testing, vortexed and visually inspected to confirm complete dissolution.

The positive control compound remdesivir was prepared as a 10 mM stock in dimethyl sulfoxide (DMSO) and stored at -20°C. Compound dilutions were prepared on the day of experimentation. A 3-fold, 8 point, DMSO dilution series of each of the four test compounds was performed initially, ranging 100 mM to 0.045 mM. An intermediate dilution series in virus growth medium (MEM supplemented with 1% (w/v) L-glutamine, 2% FBS, 8 $\mu$g/mL Tosyl Phenylalanyl Chloromethyl Ketone (TPCK)-Trypsin was generated ranging 800 $\mu$M-0.37 $\mu$M. A 50 $\mu$L volume from each compound intermediate dilution series was added to triplicate wells of the assay plate pre-seeded with Vero E6 cells. The DMSO concentration was maintained at 0.2% in the assay plate. One assay plate per compound was generated.

Similarly, remdesivir was subjected to an initial DMSO dilution series, intermediate dilution series to reduce the DMSO concentration to 0.2% in the assay plate. Remdesivir was tested at a starting concentration of 20 $\mu$M.

A 50 $\mu$L volume of SARS-CoV-2 diluted in virus growth medium to generate a multiplicity of infection (moi) of 0.05, was added to the assay plates. This moi was previously determined to provide 100% cytopathic effect (CPE) in 4 days. Virus was added to triplicate rows to assess antiviral activity and virus growth medium without virus was added to triplicate rows to assess cytotoxicity.

The percent cell protection achieved by the positive control (remdesivir) and test articles in virus-infected cells was calculated by the formula of Pauwels et al. (J. Virol. Methods 1988, 20, 309-321) as shown below:

Percent cell protection = ([ODt]virus - [ODc]virus/[ODc]mock - [ODc]virus) $\times$ 100

where:

[ODt]virus = the optical density measured in a well examining the effect of a given concentration of test article or positive control on virus-infected cells.

[ODc]virus = the optical density measured in a well examining the effect of the negative control on virus-infected cells.

[ODc]mock = the optical density measured in a well examining the effect of the negative control on mock-infected cells.

The $EC_{50}$ values were calculated from the percent cell protection results by nonlinear regression analysis using the Hill (sigmoid Emax) formula:

$$y = Min_y + (Max_y - Min_y)/1 + (EC_{50}/X)^D$$

where:

X = test or control article concentration;

Y = percent cell protection;

Min = minimum;

Max = maximum;

D = slope coefficient.

*Yield Reduction Assay*

Vero E6 cells were seeded into 96-well plates at $2 \times 10^4$ cells/well in 100 μL seeding medium (MEM supplemented with 1% (w/v) L-glutamine, 2% FBS). Plates were incubated overnight at 37°C, 5% $CO_2$.
For combination studies, compounds were added together in the deep well plate in a 1:1 ratio, then 100 μL volume of compound combination was added to cell monolayers. A 200 μL volume of virus (B3) was added to plates (moi 0.003).
After 48 h incubation, virus was harvested at each concentration and diluted 1:10 (*n* = 1) or 1:100 (*n* = 2) in virus growth media. One hundred microliters were added to triplicate wells of 96-well plates containing Vero E6 cells and serially diluted three-fold across the plate for a total of nine different virus concentrations. Six of the wells contained assay media alone (i.e., no virus) and served as controls. Plates were incubated for three days at 37°C in a humidified 5% $CO_2$ atmosphere during which time the CPE was allowed to develop. Cell monolayers were then observed microscopically with visual scoring of virus-induced CPE used as an endpoint. The $TCID_{50}$ of the virus suspension was determined using the method of Reed-Muench. (Reed, L.J.; Muench, H. A simple method of estimating fifty percent endpoints. Am. J. Hyg. 1938, 27, 493-497.) The virus yield was expressed as a percentage with respect to virus growth when no drug was added, for each concentration.

*Cytotoxicity Assay*

The cytotoxic effects of indomethacin, ketotifen, and naproxen on Vero E6 cells was tested over a 4 day or 48 h period to mimic either the antiviral assay or the yield reduction assay. Drugs were tested at the same concentration as those used in Section 2.1 and Section 2.2. Viable cells were determined by staining with MTT or crystal violet. A 100 μL volume of a 3 mg/mL solution of MTT was added to plates and incubated for 2 h at 37°C in a 5% $CO_2$ incubator. Wells were aspirated to dryness and formazan crystals solubilized by the addition of 200 μL 100% 2-Propanol at room temperature for 30 min. Absorbance was measured at 540-650 nm on a plate reader. Media were removed from cells stained with crystal violet and washed once with PBS. A 40 μL volume of 0.25% crystal violet/20% methanol stain was added to each well and incubated for 30 min at room temperature. Crystal violet stain was aspirated, monolayers washed 3-5 times with PBS and the stain solubilized with the addition of 100 μL 1% sodium dodecyl sulfate (SDS). After 30 min at room temperature, absorbance was measured at 540-650 nm on a plate reader. The 50% cytotoxic concentration ($CC_{50}$) was defined as the concentration of the test compound that reduces the absorbance of the mock infected cells by 50% of the control value. The $CC_{50}$ value was calculated as follows:

$$CC_{50} = [ODt]\text{mock}/[ODc]\text{mock}.$$

Results

Results for CPE Assay are shown in Table 1. None of the treatments exhibited antiviral activity in the 4 days CPE assay.

Table 1. $EC_{50}$ and $CC_{50}$ data from CPE assay

| Compound | $EC_{50}$ (μM) | $CC_{50}$ (μM) |
|---|---|---|
| Ketotifen | >138.6 | 138.6 |
| Naproxen | >400 | >400 |
| Indomethacin | >400 | >400 |
| Remdesivir | 2.9 | NT |

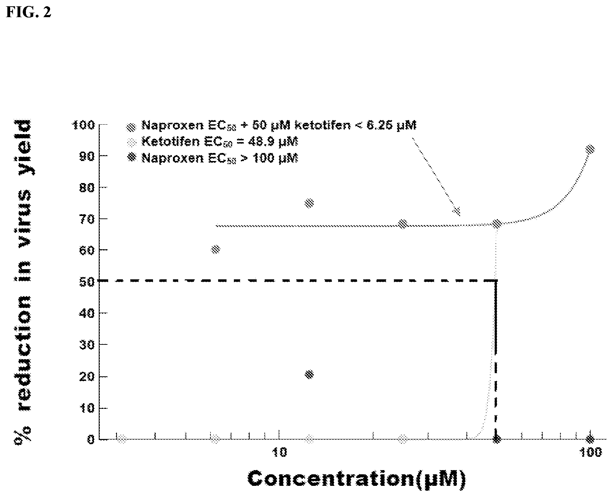

The results for ketotifen and naproxen for the Yield Reduction Assay (YRA) are summarized in Table 2 and FIG. 2. Ketotifen alone inhibited SARS-CoV-2 and exhibited an EC50 of 48.9 μM. Naproxen alone did not inhibit SARS-CoV-2 and exhibited an $EC_{50}$ of >100 μM. $EC_{50}$ is the effective concentration, i.e., the concentration at which virus infection is inhibited by 50 percent.

Table 2. EC$_{50}$ and percentage inhibition of SARS-CoV-2: 50 μM ketotifen EC$_{50}$ in combination with naproxen.

| Treatment | EC50 | Concentration Naproxen (μM) | | | | | |
|---|---|---|---|---|---|---|---|
| | | 100 | 50 | 25 | 12.5 | 6.25 | 3.125 |
| Ketotifen | 48.9 | - | 68 | 0 | 0 | 0 | 0 |
| Napr. | >100 | 0 | 0 | 0 | 21 | 0 | - |
| Napr. EC$_{50}$ + 50 μM ketotifen | <6.25 | 92 | 68 | 68 | 75 | 60 | - |

When ketotifen was added at a single concentration, naproxen's ability to reduce virus yield was enhanced. The EC$_{50}$ of naproxen was reduced to <6 μM, equating to ~17-fold greater effect in the presence of ketotifen. The dose-response curves are shown in FIG. 2. These data indicate an additive or synergistic effect.

The results for ketotifen and indomethacin are summarized in Table 3 and Table 4. Table 3 reveals that, compared to ketotifen alone (60% inhibition at EC$_{50}$), an increase in percentage inhibition of SARS-CoV-2 to 79%, 83% and 93% was observed when co-administered with 25, 50 and 100 μM indomethacin, respectively, indicating a synergistic effect.

Table 3. EC$_{50}$ and percentage inhibition of SARS-CoV-2: 50 μM ketotifen in combination with indomethacin.

| Treatment | EC$_{50}$ | Concentration Ketotifen (μM) | | | | |
|---|---|---|---|---|---|---|
| | | 50 | 25 | 12.5 | 6.25 | 3125 |
| Ketotifen | 47.1 | 60 | 0 | 0 | 0 | 0 |
| Ketotifen + 100 μM indomethacin | 48.1 | 94 | 0 | 0 | 37 | 0 |
| Ketotifen + 50 μM indomethacin | 46.5 | 97 | 0 | 0 | 0 | 37 |
| Ketotifen + 25 μM indomethacin | 42.2 | 90 | 0 | 0 | 0 | 0 |

Table 4. EC$_{50}$ and percentage inhibition of SARS-CoV-2: 100 μM indomethacin in combination with ketotifen.

| Treatment | EC50 | Concentration Indomethacin (μM) | | | | |
|---|---|---|---|---|---|---|
| | | 100 | 50 | 25 | 12.5 | 6.25 |
| Indomethacin | 100.1 | 50 | 0 | 0 | - | - |
| Indomethacin + 50 μM ketotifen | <6.25 | 93 | 83 | 79 | 75 | 60 |
| Indomethacin + 25 μM ketotifen | >100 | 0 | 0 | 0 | - | - |
| Indomethacin + 12.5 μM ketotifen | >100 | 0 | 0 | 0 | - | - |
| Indomethacin + 6.25 μM ketotifen | >100 | 37 | 0 | 0 | - | - |
| Indomethacin + 3.125 μM ketotifen | >100 | 0 | 37 | 0 | - | - |

Indomethacin alone inhibited SARS-CoV-2 and exhibited an EC$_{50}$ of 100.1 μM (see Table 4). Indomethacin did not reach an EC$_{50}$ in the presence of 50 μM ketotifen, although 60% inhibition was observed at 6.25 μM indicating that an EC$_{50}$ would probably be reached at ~3 μM had the dilution series had covered a lower range. These in vitro data indicate that a lower dose of indomethacin is required to inhibit virus growth in the presence of 50 μM of ketotifen, indicating an additive or synergistic effect. Indomethacin did not reach an EC$_{50}$ in the presence of 25 μM, 12.5, 6.25 or 3.1 μM ketotifen.

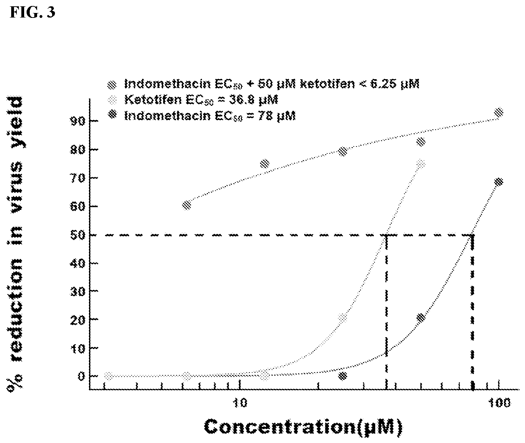

When ketotifen was added at a single concentration, indomethacin's ability to reduce virus yield was enhanced. The EC$_{50}$ of indomethacin was reduced to <6 μM, equating to ~13-fold greater effect in the presence of ketotifen. FIG. 3 shows the dose-response curves for indomethacin and ketotifen, alone and in combination. The dose-response curves suggest an additive or synergistic antiviral effect against SARS-CoV-2 when ketotifen and indomethacin are administered in combination.

The results of the cytotoxicity assessments are summarized in Table 5. The 50% cytotoxic concentration (CC$_{50}$) was defined as the compound concentration required for the reduced cell viability by 50%. No cytotoxicity was observed for indomethacin, naproxen and ketotifen at the concentrations tested.

Table 5. Percentage viability at different concentrations of ketotifen, naproxen, and indomethacin.

| Concentration (μM) | 100 | 50 | 25 | 12.5 | 6.25 | 3.13 | 1.56 | 0.78 | 0.39 | 0.20 |
|---|---|---|---|---|---|---|---|---|---|---|
| Indomethacin (%) | 143 | 143 | 125 | 124 | 124 | 117 | 111 | 106 | 98 | - |
| Naproxen (%) | 107 | 108 | 107 | 107 | 106 | 112 | 96 | 112 | 108 | - |
| Ketotifen (%) | - | 86 | 104 | 122 | 125 | 121 | 109 | 101 | 103 | 110 |

The current findings show that both combinations, ketotifen/indomethacin and ketotifen/naproxen reduce viral yield. Compared to ketotifen alone (60% inhibition at $EC_{50}$), an increase in percentage inhibition of SARS-CoV-2 to 90%, 97% and 94% was found when co-administered with 25, 50 and 100 μM indomethacin, respectively, indicating an additive or synergistic effect. The $EC_{50}$ of indomethacin was reduced to <6 μM, equating to ~13-fold greater effect in the presence of ketotifen. Compared to ketotifen alone (60% inhibition at $EC_{50}$), an increase in percentage inhibition of SARS-CoV-2 to 68%, 68% and 92% was found when co-administered with 25, 50 and 100 μM naproxen, respectively. This also indicates an additive or synergistic effect. Finally, no cytotoxic effects were observed for the concentrations of ketotifen, indomethacin, and naproxen tested.

## Conclusions

These examples show that the combination of ketotifen with indomethacin or naproxen both significantly reduces viral yield. Compared to ketotifen alone, the combination with indomethacin or naproxen has an additive or synergistic effect. No cytotoxic effects were observed for concentrations of ketotifen, naproxen, and indomethacin tested.

## Further Considerations

As used herein, the phrase "at least one of" preceding a series of items, with the term "and" or "or" to separate any of the items, modifies the list as a whole, rather than each member of the list (i.e., each item). The phrase "at least one of" does not require selection of at least one of each item listed; rather, the phrase allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

As used herein, the term "about", unless the context requires otherwise, means ±10% of an associated numerical value.

As used herein, the term "comprising" indicates the presence of the specified feature, but allows for the possibility of other features, unspecified. This term does not imply any particular proportion of the specified features. Variations of the word "comprising," such as "comprise" and "comprises," have correspondingly similar meanings.

The word "exemplary" is used herein to mean "serving as an example, instance, or illustration." Any embodiment described herein as "exemplary" is not necessarily to be construed as preferred or advantageous over other embodiments.

A reference to an element in the singular is not intended to mean "one and only one" unless specifically stated, but rather "one or more." All structural and functional equivalents to the elements of the various configurations described throughout this disclosure that are known or later come to be known to those of ordinary skill in the art are expressly incorporated herein by reference and intended to be encompassed by the subject technology.

## Claims

1. A non-steroidal anti-inflammatory drug (NSAID) and ketotifen for use in treating a beta coronavirus infection in a subject in need thereof, wherein the NSAID is selected from naproxen or indomethacin.

2. The non-steroidal anti-inflammatory drug (NSAID) and ketotifen for use of claim 1, wherein the subject is a human.

3. The non-steroidal anti-inflammatory drug (NSAID) and ketotifen for use of claim 1 or 2, wherein the beta coronavirus is SARS-CoV-2, optionally wherein the subject has tested positive for SARS-CoV-2, and optionally wherein the SARS-CoV-2 is alpha, beta, gamma, delta, or lambda variant.

4. The non-steroidal anti-inflammatory drug (NSAID) and ketotifen for use of any one of claims 1 to 3, wherein the subject is over the age of 65; and/or is immunocompromised, optionally due to therapy with an immunosuppressant; and/or has a chronic health condition, optionally wherein the chronic health condition is selected from one or more of obesity; diabetes mellitus; AIDS; a chronic lung condition optionally selected from asthma, COPD, pulmonary fibrosis, cystic fibrosis, pulmonary hypertension, emphysema, and bronchitis; neutrophilia; history of myocardial infarction or stroke; coronary artery disease; congestive heart failure; cardiomyopathy; dementia or Alzheimer's disease; chronic kidney or liver disease; organ transplantation; and autoimmune disease.

5. The non-steroidal anti-inflammatory drug (NSAID) and ketotifen for use of claims 3 or 4, wherein the subject has tested positive for SARS-CoV2 and the subject is asymptomatic for SARS-CoV-2 associated disease; or wherein the subject has tested positive for SARS-CoV2 and the subject is experiencing one or more symptoms of COVID-19, optionally wherein the symptoms include one or more of congestion, cough, fever, loss of taste, loss of smell, muscle pain, joint pain (myalgia), fatigue, sore throat, headache, myocarditis, coagulopathy, renal injury or failure, liver damage or failure, neurological impairment, hypoxia, pneumonia with or without hypoxia, acute respiratory syndrome, or multi-organ failure, and optionally wherein the subject has symptoms of hypoxia and/or acute respiratory distress syndrome, or the subject has chronic headache and/or fatigue, or the subject has long COVID.

6. The non-steroidal anti-inflammatory drug (NSAID) and ketotifen for use of any one of claims 1 to 5, wherein the NSAID and ketotifen are to be administered in separate compositions.

7. The non-steroidal anti-inflammatory drug (NSAID) and ketotifen for use of any one of claims 1 to 5, wherein the NSAID and ketotifen are to be administered in the same composition.

8. The non-steroidal anti-inflammatory drug (NSAID) and ketotifen for use of claim 7, wherein the composition is administered orally, intravenously, solution or powder aerosol for inhalation, intramuscularly, subcutaneously, transdermally, or intranasally, optionally wherein the composition is administered by a nebulizer.

9. The non-steroidal anti-inflammatory drug (NSAID) and ketotifen for use of claim 7, wherein the composition is in the form of a table, capsule, a lozenge or chewing gum.

10. The non-steroidal anti-inflammatory drug (NSAID) and ketotifen for use of claim 8 or 9, wherein the composition consists essentially of the NSAID and ketotifen as active pharmaceutical ingredients, optionally wherein the composition consists of the NSAID and ketotifen as active pharmaceutical ingredients.

11. The non-steroidal anti-inflammatory drug (NSAID) and ketotifen for use of any of claims 8 to 10, wherein the composition further comprises a pharmaceutical carrier, a pharmaceutical excipient, a sweetening agent, a flavoring agent, a taste masking ingredient, a diluent, alum, a stabilizer, a buffer, a coloring agent, a breath neutralizer, an emulsifying agent, a suspending agent, a nebulizing agent and combinations thereof.

12. The non-steroidal anti-inflammatory drug (NSAID) and ketotifen for use of any one of claims 1 to 11, wherein the NSAID and ketotifen are administered before the onset of COVID symptoms, or wherein the NSAID and ketotifen are administered after the onset of COVID symptoms, optionally wherein the NSAID and ketotifen are administered at the first appearance of a symptom of COVID-19, optionally during an early infection phase.

13. The non-steroidal anti-inflammatory drug (NSAID) and ketotifen for use of any one of claims 1 to 12, wherein the NSAID and ketotifen are administered at least once daily, optionally wherein the NSAID and ketotifen are administered from about once daily to about three times daily, and optionally wherein the NSAID and ketotifen are administered for at least one week or for at least two weeks, or for at least three weeks, or about one month.

14. The non-steroidal anti-inflammatory drug (NSAID) and ketotifen for use of any one of claims 1 to 13, wherein a daily dose of indomethacin is about 20 mg to about 300 mg, about 40 mg to about 200 mg, or about 75 mg to about 150 mg, or wherein a daily dose of naproxen is about 100 mg to about 1600 mg, about 200 mg to about 1400 mg, about 400 mg to about 1200 mg, about 600 mg to about 1000 mg, or about 700 mg to about 900 mg.

15. The non-steroidal anti-inflammatory drug (NSAID) and ketotifen for use of claim 14, wherein a daily dose of ketotifen is about 1 mg to about 10 mg, or about 1 mg to about 6 mg, or about 2 mg to about 5 mg, or about 3 mg to about 4 mg.

16. The non-steroidal anti-inflammatory drug (NSAID) and ketotifen for use of claim 14 or 15, wherein the NSAID and the ketotifen are combined in a unit dose, optionally wherein the NSAID is indomethacin, the amount of indomethacin in the unit dose is about 50 mg, the amount of ketotifen is about 1 mg, and the unit dose is administered two or three times per day.

**Patentansprüche**

1. Ein nichtsteroidales Antirheumatikum (NSAR) und Ketotifen zur Verwendung bei der Behandlung einer Beta-Coronavirus-Infektion bei einem Subjekt, das eine solche Behandlung benötigt, wobei das NSAR aus Naproxen oder Indomethacin ausgewählt ist.

2. Das nichtsteroidale Antirheumatikum (NSAR) und Ketotifen zur Verwendung nach Anspruch 1, wobei das Subjekt ein Mensch ist.

3. Das nichtsteroidale Antirheumatikum (NSAR) und Ketotifen zur Verwendung nach Anspruch 1 oder 2, wobei das Beta-Coronavirus SARS-CoV-2 ist, optional wobei das Subjekt positiv auf SARS-CoV-2 getestet wurde und optional wobei das SARS-CoV-2 eine Alpha-, Beta-, Gamma-, Delta- oder Lambda-Variante ist.

4. Das nichtsteroidale Antirheumatikum (NSAR) und Ketotifen zur Verwendung nach einem der Ansprüche 1 bis 3, wobei das Subjekt über 65 Jahre alt ist und/oder immungeschwächt ist, optional aufgrund einer Therapie mit einem Immunsuppressivum, und/oder eine chronische Erkrankung hat, optional wobei die chronische Erkrankung aus einer oder mehreren von Adipositas, Diabetes mellitus, AIDS; einer chronischen Lungenerkrankung, die optional aus Asthma, COPD, Lungenfibrose, Mukoviszidose, pulmonaler Hypertonie, Emphysem und Bronchitis ausgewählt ist; Neutrophilie; einer Vorgeschichte von Myokardinfarkt oder Schlaganfall; koronarer Herzkrankheit; kongestiver Herzinsuffizienz; Kardiomyopathie; Demenz oder Alzheimer-Krankheit; chronischer Nieren- oder Lebererkrankung; Organtransplantation und Autoimmunerkrankung, ausgewählt ist.

5. Das nichtsteroidale Antirheumatikum (NSAR) und Ketotifen zur Verwendung nach Anspruch 3 oder 4, wobei das Subjekt positiv auf SARS-CoV2 getestet wurde und das Subjekt asymptomatisch für eine mit SARS-CoV-2 assoziierte Erkrankung ist; oder wobei das Subjekt positiv auf SARS-CoV2 getestet wurde und das Subjekt eines oder mehrere Symptome von COVID-19 aufweist, optional wobei die Symptome eines oder mehrere von Verstopfung, Husten, Fieber, Verlust des Geschmackssinns, Verlust des Geruchssinns, Muskelschmerzen, Gelenkschmerzen (Myalgie), Müdigkeit, Halsschmerzen, Kopfschmerzen, Myokarditis, Koagulopathie, Nierenverletzung oder -versagen, Leberschädigung oder - versagen, neurologische Beeinträchtigung, Hypoxie, Lungenentzündung mit oder ohne Hypoxie, akutes respiratorisches Syndrom oder Multiorganversagen umfassen, und optional wobei das Subjekt Symptome von Hypoxie und/oder akutem Atemnotsyndrom aufweist oder das Subjekt chronische Kopfschmerzen und/oder Müdigkeit aufweist oder das Subjekt Long COVID aufweist.

6. Das nichtsteroidale Antirheumatikum (NSAR) und Ketotifen zur Verwendung nach einem der Ansprüche 1 bis 5, wobei das NSAR und Ketotifen in getrennten Zusammensetzungen zu verabreichen sind.

7. Das nichtsteroidales Antirheumatikum (NSAR) und Ketotifen zur Verwendung nach einem der Ansprüche 1 bis 5, wobei das NSAR und Ketotifen in derselben Zusammensetzung zu verabreichen sind.

8. Das nichtsteroidale Antirheumatikum (NSAR) und Ketotifen zur Verwendung nach Anspruch 7, wobei die Zusammensetzung oral, intravenös, als Lösung oder Pulver-Aerosol zur Inhalation, intramuskulär, subkutan, transdermal oder intranasal verabreicht wird, optional wobei die Zusammensetzung mittels eines Verneblers, verabreicht wird.

9. Das nichtsteroidale Antirheumatikum (NSAR) und Ketotifen zur Verwendung nach Anspruch 7, wobei die Zusammensetzung in Form einer Tablette, Kapsel, Lutschtablette oder Kaugummi ist.

10. Das nichtsteroidale Antirheumatikum (NSAR) und Ketotifen zur Verwendung nach Anspruch 8 oder 9, wobei die Zusammensetzung im Wesentlichen aus dem NSAR und Ketotifen als pharmazeutisch wirksamen Bestandteilen besteht, optional wobei die Zusammensetzung aus dem NSAR und Ketotifen als pharmazeutisch wirksamen Bestandteilen besteht.

11. Das nichtsteroidale Antirheumatikum (NSAR) und Ketotifen zur Verwendung nach einem der Ansprüche 8 bis 10, wobei die Zusammensetzung ferner einen pharmazeutischen Träger, einen pharmazeutischen Hilfsstoff, ein Süßungsmittel, einen Aromastoff, einen Geschmacksmaskierungsstoff, ein Verdünnungsmittel, Alaun, einen Stabilisator, einen Puffer, ein Färbemittel, einen Atemerfrischer, ein Emulgiermittel, ein Suspensionsmittel, ein Zerstäubungsmittel und Kombinationen davon umfasst.

12. Das nichtsteroidale Antirheumatikum (NSAR) und Ketotifen zur Verwendung nach einem der Ansprüche 1 bis 11, wobei das NSAR und Ketotifen vor dem Auftreten von COVID-Symptomen verabreicht werden oder wobei das NSAR und Ketotifen nach dem Auftreten von COVID-Symptomen verabreicht werden, optional wobei das NSAR und Ketotifen beim ersten Auftreten eines Symptoms von COVID-19 verabreicht werden, optional während einer frühen Infektionsphase.

13. Das nichtsteroidale Antirheumatikum (NSAR) und Ketotifen zur Verwendung nach einem der Ansprüche 1 bis 12, wobei das NSAR und Ketotifen mindestens einmal täglich verabreicht werden, optional wobei das NSAR und Ketotifen etwa einmal täglich bis etwa dreimal täglich verabreicht werden und optional wobei das NSAR und Ketotifen mindestens eine Woche oder mindestens zwei Wochen oder mindestens drei Wochen oder etwa einen Monat lang verabreicht werden.

14. Das nichtsteroidale Antirheumatikum (NSAR) und Ketotifen zur Verwendung nach einem der Ansprüche 1 bis 13, wobei eine Tagesdosis von Indomethacin etwa 20 mg bis etwa 300 mg, etwa 40 mg bis etwa 200 mg oder etwa 75 mg bis etwa 150 mg beträgt, oder wobei eine Tagesdosis von Naproxen etwa 100 mg bis etwa 1600 mg, etwa 200 mg bis etwa 1400 mg, etwa 400 mg bis etwa 1200 mg, etwa 600 mg bis etwa 1000 mg oder etwa 700 mg bis etwa 900 mg beträgt.

15. Das nichtsteroidale Antirheumatikum (NSAR) und Ketotifen zur Verwendung nach Anspruch 14, wobei eine Tagesdosis von Ketotifen etwa 1 mg bis etwa 10 mg, oder etwa 1 mg bis etwa 6 mg, oder etwa 2 mg bis etwa 5 mg oder etwa 3 mg bis etwa 4 mg beträgt.

16. Das nichtsteroidale Antirheumatikum (NSAR) und Ketotifen zur Verwendung nach Anspruch 14 oder 15, wobei das NSAR und das Ketotifen in einer Einzeldosis kombiniert sind, optional wobei das NSAR Indomethacin ist, die Menge an Indomethacin in der Einzeldosis etwa 50 mg beträgt, die Menge an Ketotifen etwa 1 mg beträgt und die Einzeldosis zwei- oder dreimal täglich verabreicht wird.

## Revendications

1. Un médicament anti-inflammatoire non stéroïdien (AINS) et kétotifène pour utilisation dans le traitement d'une infection par le bêta-coronavirus chez un sujet qui en a besoin, dans lequel l'AINS est choisi parmi le naproxène ou l'indométacine.

2. Le médicament anti-inflammatoire non stéroïdien (AINS) et kétotifène pour utilisation selon la revendication 1, dans lequel le sujet est un humain.

3. Le médicament anti-inflammatoire non stéroïdien (AINS) et kétotifène pour utilisation selon la revendication 1 ou 2, dans lequel le bêta-coronavirus est le SARS-CoV-2, facultativement dans lequel le sujet a été testé positif au SARS-CoV-2, et facultativement dans lequel le SARS-CoV-2 est un variant alpha, bêta, gamma, delta ou lambda.

4. Le médicament anti-inflammatoire non stéroïdien (AINS) et kétotifène pour utilisation selon l'une quelconque des revendications 1 à 3, dans lequel le sujet est âgé de plus de 65 ans ; et/ou est immunodéprimé, facultativement en raison d'une thérapie avec un immunosuppresseur ; et/ou présente un problème de santé chronique, facultativement dans lequel le problème de santé chronique est choisi parmi une ou plusieurs de l'obésité ; le diabète sucré ; le SIDA ; une affection pulmonaire chronique facultativement choisie parmi l'asthme, la BPCO, la fibrose pulmonaire, la fibrose kystique, l'hypertension pulmonaire, l'emphysème et la bronchite ; une neutrophilie ; des antécédents d'infarctus du myocarde ou d'AVC ; une coronaropathie ; une insuffisance cardiaque congestive ; une cardiomyopathie ; une démence ou la maladie d'Alzheimer ; une maladie rénale ou hépatique chronique ;une transplantation d'organe ; et une maladie auto-immune.

5. Le médicament anti-inflammatoire non stéroïdien (AINS) et kétotifène pour utilisation selon les revendications 3 ou 4, dans lequel le sujet a été testé positif au SARS-CoV2 et le sujet est asymptomatique pour une maladie associée au SARS-CoV-2 ; ou dans lequel le sujet a testé positif au SARS-CoV2 et le sujet présente un ou plusieurs symptômes de la COVID-19, facultativement dans lequel les symptômes incluent un ou plusieurs symptômes parmi congestion, toux, fièvre, perte de goût, perte d'odorat, douleurs musculaires, douleurs articulaires (myalgie), fatigue, maux de gorge, maux de tête, myocardite, coagulopathie, lésion ou insuffisance rénale, lésion ou insuffisance hépatique, atteinte neurologique, hypoxie, pneumonie avec ou sans hypoxie, syndrome respiratoire aigu ou insuffisance multiorganique, et facultativement dans lequel le sujet présente des symptômes d'hypoxie et/ou un syndrome de détresse respiratoire aiguë, ou le sujet présente des céphalées et/ou de la fatigue chroniques, ou le sujet présente une COVID de longue durée.

6. Le médicament anti-inflammatoire non stéroïdien (AINS) et kétotifène pour utilisation selon l'une quelconque des revendications 1 à 5, dans lequel l'AINS et le kétotifène doivent être administrés dans des compositions distinctes.

7. Le médicament anti-inflammatoire non stéroïdien (AINS) et kétotifène pour utilisation selon l'une quelconque des revendications 1 à 5, dans lequel l'AINS et le kétotifène doivent être administrés dans la même composition.

8. Le médicament anti-inflammatoire non stéroïdien (AINS) et kétotifène pour utilisation selon la revendication 7, dans lequel la composition est administrée par voie orale, par voie intraveineuse, sous forme de solution ou de poudre en aérosol pour inhalation, par voie intramusculaire, sous-cutanée, transdermique ou intranasale, facultativement dans lequel la composition est administrée par un nébuliseur.

9. Le médicament anti-inflammatoire non stéroïdien (AINS) et kétotifène pour utilisation selon la revendication 7, dans lequel la composition se présente sous la forme d'un comprimé, d'une gélule, d'une pastille ou d'un chewing-gum.

10. Le médicament anti-inflammatoire non stéroïdien (AINS) et kétotifène pour utilisation selon la revendication 8 ou 9, dans lequel la composition consiste essentiellement en l'AINS et le kétotifène en tant qu'ingrédients pharmaceutiques actifs, facultativement dans lequel la composition consiste en l'AINS et le kétotifène en tant qu'ingrédients pharmaceutiques actifs.

11. Le médicament anti-inflammatoire non stéroïdien (AINS) et kétotifène pour utilisation selon l'une quelconque des revendications 8 à 10, dans lequel la composition comprend en outre un vecteur pharmaceutique, un excipient pharmaceutique, un édulcorant, un aromatisant, un ingrédient de masquage du goût, un diluant, de l'alun, un stabilisant, un tampon, un agent colorant, un neutralisant d'haleine, un agent émulsifiant, un agent suspenseur, un agent nébuliseur, et des combinaisons de ceux-ci.

12. Le médicament anti-inflammatoire non stéroïdien (AINS) et kétotifène pour utilisation selon l'une quelconque des revendications 1 à 11, dans lequel l'AINS et le kétotifène sont administrés avant le commencement des symptômes de la COVID, ou dans lequel l'AINS et le kétotifène sont administrés après le commencement des symptômes de la COVID, facultativement dans lequel l'AINS et le kétotifène sont administrés à la première apparition d'un symptôme de la COVID-19, facultativement pendant une phase précoce d'infection.

13. Le médicament anti-inflammatoire non stéroïdien (AINS) et kétotifène pour utilisation selon l'une quelconque des revendications 1 à 12, dans lequel l'AINS et le kétotifène sont administrés au moins une fois par jour, facultativement dans lequel l'AINS et le kétotifène sont administrés d'environ une fois par jour à environ trois fois par jour, et facultativement dans lequel l'AINS et le kétotifène sont administrés pendant au moins une semaine ou pendant au moins deux semaines, ou pendant au moins trois semaines, ou environ un mois.

14. Le médicament anti-inflammatoire non stéroïdien (AINS) et kétotifène pour utilisation selon l'une quelconque des revendications 1 à 13, dans lequel une dose quotidienne d'indométacine est d'environ 20 mg à environ 300 mg, d'environ 40 mg à environ 200 mg, ou d'environ 75 mg à environ 150 mg, ou dans lequel une dose quotidienne de naproxène est d'environ 100 mg à environ 1600 mg, d'environ 200 mg à environ 1400 mg, d'environ 400 mg à environ 1200 mg, d'environ 600 mg à environ 1000 mg, ou d'environ 700 mg à environ 900 mg.

15. Le médicament anti-inflammatoire non stéroïdien (AINS) et kétotifène pour utilisation selon la revendication 14, dans lequel une dose quotidienne de kétotifène est d'environ 1 mg à environ 10 mg, ou d'environ 1 mg à environ 6 mg, ou d'environ 2 mg à environ 5 mg, ou d'environ 3 mg à environ 4 mg.

16. Le médicament anti-inflammatoire non stéroïdien (AINS) et kétotifène pour utilisation selon la revendication 14 ou 15, dans lequel l'AINS et le kétotifène sont combinés en une dose unitaire, facultativement dans lequel l'AINS est l'indométacine, la quantité d'indométacine dans la dose unitaire est d'environ 50 mg, la quantité de kétotifène est d'environ 1 mg, et la dose unitaire est administrée deux ou trois fois par jour.

FIG. 1

**FIG. 2**

**FIG. 3**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Virtual screening of approved drugs as potential SARS-CoV-2 main protease inhibitors. **JIMENEZ-ALBERTO ALICIA et al.** Computational Biology and Chem.. Elsevier, 2020, vol. 88
- **NAPOLI PIETRO EMANUELE et al.** A Panel of Broad-Spectrum Antivirals in Topical Ophthalmic Medications from the Drug Repurposing Approach during and after the Coronavirus Disease 2019 Era. *Journal of Clinical Medicine*, 2020, vol. 9 (8)
- **PAUWELS et al.** *J. Virol. Methods*, 1988, vol. 20, 309-321
- **REED, L.J.** ; **MUENCH, H**. A simple method of estimating fifty percent endpoints. *Am. J. Hyg.*, 1938, vol. 27, 493-497